(19) Europäisches Patentamt
European Patent Office
Office européen des brevets

(11) Publication number: 0 143 613
B1

# (12) EUROPEAN PATENT SPECIFICATION

(45) Date of publication of patent specification: 08.02.89

(51) Int. Cl.$^4$: C 07 C 118/02, C 07 C 119/042

(21) Application number: 84308106.8

(22) Date of filing: 22.11.84

## (54) Production of isocyanate compounds.

(30) Priority: 28.11.83 JP 225226/83
15.11.84 JP 241876/84

(43) Date of publication of application:
05.06.85 Bulletin 85/23

(45) Publication of the grant of the patent:
08.02.89 Bulletin 89/06

(84) Designated Contracting States:
AT BE CH DE FR GB IT LI NL

(56) References cited:
DIE MAKROMOLEKULARE CHEMIE, vol. 131, no. 3199, 1970, pages 247-257, Hüthig & Wepf Verlag, Basel, CH; H. DIEFENBACH et al.: "Über ungesättigte Harnstoff- und Oxalsäurederivate"

"Methoden der Organischen Chemie", vol. VII/2c, Ketone, part III, 4th edition, 1977, pages 2137-2138, Georg Thieme Verlag, Stuttgart, DE;

(73) Proprietor: NIPPON PAINT CO., LTD.
1-2, Oyodokita 2-chome Oyodo-ku
Osaka-shi Osaka-fu (JP)

(72) Inventor: Urano, Satoshi
A36-301, No. 6, Otokoyamakohro
Yawata-shi Kyoto-fu (JP)
Inventor: Mizuguchi, Ryuzo
C410, No. 8, Yawatakitaura
Yawata-shi Kyoto-fu (JP)

(74) Representative: Perry, Robert Edward et al
GILL JENNINGS & EVERY 53-64 Chancery Lane
London WC2A 1HN (GB)

(56) References cited:
M. FIESER et al.: "Reagents for organic synthesis", vol. 6, 1977, pages 411,412, John Wiley & Sons, New York, US;

S.PATAI: " The chemistry of cyanates and their thioderivatives", part 2, chapter 17, 1977, pp. 665-679 and 792-805

EP 0 143 613 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to the production of isocyanate compounds. More particularly, it relates to the production of alkenoyl isocyanates of the formula

$$CH_2{=}CR{-}CO{-}N{=}C{=}O \qquad (I)$$

wherein R is H or $C_{1-5}$ alkyl, e.g. via haloalkanoyl isocyanates of the formula

$$XCH_2{-}CHR{-}CO{-}N{=}C{=}O \qquad (II)$$

wherein X is a halogen atom and R is as defined above.

$C_{1-5}$ alkyl is preferably $C_{1-3}$ alkyl, e.g. methyl, ethyl or propyl, more preferably methyl. The term "halogen" is intended to mean chlorine, bromine, iodine or fluorine, preferably chlorine or bromine, and particularly chlorine.

In general, compounds having an isocyanate group are widely used in the field of polymer chemistry because of their high reactivity. Those compounds having both a polymerizable double bond and an isocyanate group are expected to be particularly useful, because the double bond and the isocyanate group can respectively participate in various reactions depending upon different reaction mechanisms. Specific examples of such compounds are vinyl isocyanate (Angew. Chem., Int. Ed., *18*, 319 (1979)), isocyanatoethyl methacrylate (Japanese Patent Publn. (unexamined) No. 5921/79) and acryloyl and methacryloyl isocyanates (Chem. Ber., *84*, 4 (1951)).

Acryloyl isocyanate (I: R=H) and methacryloyl isocyanate (I: $R{=}CH_3$) are particularly interesting in that the reactivities of the polymerizable double bond and the isocyanate group are enhanced by the carbonyl group present between them.

Alkenoyl isocyanates (I) can undertake various reactions such as radical polymerization, anion polymerization, dimerization, trimerization, polar addition, and addition of active hydrogen based on the $CH_2{=}CR{-}C({=}O){-}$ partial structure (i.e. having conjugated double bonds) and/or on the $-C({=}O){-}N{=}C{=}O$ partial structure (i.e. acyl isocyanate structure).

The alkenoyl isocyanates (I) were originally prepared by reacting the corresponding alkenoyl chlorides with silver isocyanate (Chem. Ber., *84*, 4 (1951)). Apparently, this process is industrially disadvantageous in using an expensive reagent such as silver isocyanate. US—A—3155700 discloses using isocyanic acid in place of silver isocyanate. However, isocyanic acid is produced by thermal decomposition of its trimer, i.e. isocyanuric acid, at as a high temperature as 620°C. In addition, isocyanic acid is a gaseous material. Accordingly, special apparatus is needed, and handling of the gaseous material is troublesome.

In 1962 to 1965, Speziale *et al* developed a process for the production of acyl isocyanates by reacting amides with oxalyl chloride (J. Org. Chem., *27*, 3742 (1962); ibid., *23*, 1805 (1963), ibid., *30*, 4306 (1965). When this process is applied to aromatic amides, the desired acyl isocyanates are usually obtained in good yields. However, its application to aliphatic amides cannot usually afford the desired acyl isocyanate in noticeable yields.

In fact, the present inventors have reacted acrylamide with oxalyl chloride according to the Speziale *et al* procedure, but the desired acryloyl isocyanate (I: R=H) could not be recovered from the reaction mixture. Although efforts to obtain the desired acryloyl isocyanate were made under various reaction conditions, no success was achieved.

Then, the present inventors applied the Speziale *et al* procedure to methacrylamide in place of acrylamide, and found surprisingly that methacryloyl isocyanate (I: $R{=}CH_3$) can be recovered from the reaction mixture in a noticeable yield. Study of the reaction mixture revealed that it included, in addition to methylacryloyl isocyanate, a considerable amount of α-methyl-β-chloropropionyl isocyanate (II: $R{=}CH_3$; X=Cl).

With this knowledge, the mixture of reaction between acrylamide and oxalyl chloride was then carefully examined. Its major component was confirmed to be β - chloropropionyl isocyanate (II: R=H; X=Cl).

Diefenbach *et al*, Die Makromolekulare Chemie *131* (1970) 247—257, imply that (meth)acryloyl isocyanate (I: $R{=}CH_3$, H) is obtained as an intermediate when (meth)acrylamide is reacted with oxalyl chloride, to obtain II ($R{=}CH_3$, H).

According to a first aspect of the present invention, a process for preparing an alkenoyl isocyanate of the formula

$$CH_2{=}CR{-}CO{-}NCO \qquad (I:\ R{=}C_{1-5}\ \text{alkyl})$$

comprises reacting an acrylamide of the formula

$$CH_2{=}CR{-}CONH_2 \qquad (III:\ R{=}C_{1-5}\ \text{alkyl})$$

with an oxalyl halide of the formula

$(COX)_2$ (IV: X=halogen)

at −50 to +150°C; and recovering the alkenoyl isocyanate from the resultant reaction mixture by distillation.

According to a second aspect of the present invention, a process for preparing acryloyl isocyanate (I: R=H) which comprises reacting acrylamide (III: R=H) with an oxalyl halide as defined above (IV: X=halogen), at −50 to +150°C; and reacting the resultant β - halopropionyl isocyanate (II: R=H, X=halogen) with a hydrogen halide-eliminating reagent, at −50 to +200°C.

It has been confirmed that the hydrogen halide elimination is reversible. For example, the reaction of acryloyl isocyanate (I: R=H) or methacryloyl isocyanate (I: $R=CH_3$) with hydrogen chloride gives β - chloropropionyl isocyanate (II: R=H; X=Cl) or α - methyl - β - chloropropionyl isocyanate (II: $R=CH_3$; X=Cl) respectively.

The molar ratio of the acrylamide (III) to the oxalyl halide (IV) to be reacted is usually 10—0.1:1, preferably 1—0.5:1. No reaction solvent is necessary, but its use is normally preferred. The reaction solvent may be any insert solvent, particularly a halogenated hydrocarbon, of which specific examples are carbon tetrachloride, chloroform, dichloromethane, 1,1- or 1,2 - dichloroethane, 1,6 - dichlorohexane, 1,5 - dichloropentane, 1,2-, 1,3- or 2,2 - dichloropropane, 1,1,1- or 1,1,2 - trichloroethane, 1,1,1,2- or 1,1,2,2 - tetrachloroethane, 1,4- or 2,3 - dichlorobutane, 1- or 2 - chlorobutane, chlorobenzene, chlorocyclohexane, tetrachloroethylene, trichloroethylene, pentachloroethane, chloropropane, 1,2 - dichloroethylene, a dichlorobenzene, a chlorotoluene, 1,2,4 - trichlorobenzene, bromobenzene, bromoethane, 1- or 2 - bromopropane, 1- or 2 - bromobutane, 1- or 2 - bromopentane, a bromotoluene, bromocyclohexane, bromochloroethane and 1 - bromohexane. The reaction temperature is from −50 to 150°C, preferably from −30 to 100°C.

In the above reaction, the haloalkanoyl isocyanate (II) is the sole major reaction product when R=H. When R=alkyl, the alkenoyl isocyanate (I) and the haloalkanoyl isocyanate (II) are the major reaction products. Under usual reaction conditions, the amount of each is nearly the same; the tendency is for the amount of the alkenoyl isocyanate (I) to be greater at a lower reaction temperature. Separation of the alkenoyl isocyanate (I) from the haloalkanoyl isocyanate (II) may be accomplished with ease by distillation under atmospheric or reduced pressure.

Preferably, the haloalkanoyl isocyanate (II: R=alkyl) is recovered from the reaction mixture separately from the alkenyl isocyanate (I: R=alkyl), e.g. by a per se conventional separation procedure such as distillation under atmospheric or reduced pressure, and is then reacted with a hydrogen halide-eliminating agent at −50 to +200°C, to give the alkenoyl isocyanate (I). This reaction is always necessary when R=H.

The hydrogen halide-eliminating agent may be not only a hydrogen halide-eliminating agent in a strict sense, i.e. one used in an amount at least theoretically equimolar with respect to the haloalkanoyl isocyanate (II), but also a hydrogen halide-eliminating catalyst, which may be employed in an amount smaller than equimolar. Specific examples of the hydrogen halide-eliminating agent are amines such as triethylamine, 1,8 - diazabicyclo[5.4.0]undecene - 7, pyridine or quinoline, alkali metal or alkaline earth metal hydroxides such as sodium hydroxide, potassium hydroxide or magnesium hydroxide, metal oxides such as copper oxide, magnesium oxide, calcium oxide, alumina or iron oxide, metal complexes such as $(Ph_3P)_2Ru(CO)_3$ or $(PhP)_3Pt$ (wherein Ph is phenyl), metal halides such as lithium chloride, titanium chloride, aluminium chloride or sodium chloride, metal salts such as zinc naphthenate, nickel acetate, barium sulfate or potassium phosphate, metal alkoxides such as potassium t-butoxide, sodium ethoxide or sodium isopropoxide, synthetic zeolites such as molecular sieve or microporous glass, boric acid, oxirane, metallic zinc and triphenylphosphine. Amines, metal oxides, metal halides, synthetic zeolites and triphenylphosphine are particularly preferred.

The reaction is normally effected using the hydrogen halide-eliminating agent in an amount of 0.1 to 100 mol, preferably 0.1 to 10 mol, per mol of the haloalkyanoyl isocyanate (II), in the presence or absence of an appropriate inert solvent. Specific examples of the inert solvent are aliphatic hydrocarbons (e.g. pentane, hexane, heptane), aromatic hydrocarbons (e.g. benzene, toluene, xylene), alicyclic hydrocarbons (e.g. cyclohexane, methylcyclohexane, decalin), other hydrocarbon solvents (e.g. petroleum ether, petroleum benzin), halogenated hydrocarbons (e.g. carbon tetrachloride, chloroform, 1,2 - dichloroethane), ethers (e.g. ethyl ether, isopropyl ether, anisole, dioxane, tetrahydrofuran), ketones (e.g. acetone, methyl ethyl ketone, methyl isobutyl ketone, cyclohexanone, acetophenone, isophorone), esters (e.g. ethyl acetate, butyl acetate), acetonitrile, dimethyl formamide and dimethylsulfoxide. The reaction temperature is −50 to 200°C, preferably from 0 to 150°C.

Recovery of the reaction product from the reaction mixture may be accomplished by a per se conventional separation procedure such as distillation under atmospheric or reduced pressure.

In any of the reactions described above, a small amount of a polymerization inhibitor may be incorporated into the reaction system or the reaction mixture for prevention of the unnecessary polymerization at the double bond. Examples of the polymerization inhibitor are hydroquinone, p - methoxyphenol, 2,6 - di - t - butyl - 4 - methylphenol, 4 - t - butylcatechol, bisdihydroxybenzylbenzene, 2,2′ - methylene - bis(6 - t - butyl - 3 - methylphenol), 4,4′ - butylidene - bis(6 - t - butyl - 3 - methylphenol), 4,4′ - thiobis(6 - t - butyl - 3 - methylphenol), p - nitrosophenol, diisopropylxanthogenesulfide, N - nitrosophenylhydroxylamine ammonium salt, 1,1 - diphenyl - 2 - picrylhydrazil, 1,3,5 -

triphenylpheldazyl, 2,6 - di - t - butyl - α - (3,5 - di - t - butyl - 4 - oxo - 2,5 - cyclohexaden - 1 - ylidene) - p - trioxy, 2,2,6,6 - tetramethyl - 4 - piperidone - 1 - oxil, dithiobenzoyl sulfide, p,p' - ditolyl trisulfide, p,p' - ditolyl tetrasulfide, dibenzyl tetrasulfide and tetraethylthiuram disulfide.

The alkenoyl isocyanate (I) and the haloalkanoyl isocyanate (II) are, in general, liquids which are stable at room temperature. They can therefore be handled with ease. They are soluble in various organic solvents and can be used in their solution form. When allowed to stand in air, they readily react with moisture in the air to give the corresponding amides. This property is desirable, from the viewpoint of avoiding environmental pollution. Their double bond or halogen atom is very reactive and can be readily reacted with other compounds to give isocyanate derivatives.

Alkenoyl isocyanates (I) exert strong antimicrobial activity in the gaseous state and are therefore useful as antimicrobial agents. Furthermore, alkenoyl isocyanates (I) can participate in various chemical reactions due to the functional groups present therein and therefore can be used for the production of starting materials and intermediates in the fields of pharmaceuticals, agro-chemicals, dyestuffs, etc. They also find wide use as monomers for the production of various polymers. For instance, their copolymerization with styrene, alkyl acrylates, or alkyl methacrylates affords varnish resins; their copolymerization with other monomers affords polymers useful as dyestuffs, adhesives, dipping agents, foaming agents and fibre-treating agents.

In general, the isocyanate compounds wherein R is alkyl, e.g. methacryloyl isocyanate and α - methyl - β - chloropropionyl isocyanate, are more stable than the corresponding isocyanate compounds wherein R is H, e.g. acryloyl isocyanate and β - chloropropionyl isocyanate, particularly when heated.

The following Examples illustrate the invention. Percentages are by weight.

Example 1

To a suspension of methacrylamide (17.9 g) and hydroquinone (0.18 g) in chloroform (100 ml), a solution of oxalyl chloride (20 ml) in chloroform (15 ml) was dropwise added while ice-cooling at 0°C under a stream of nitrogen. The resultant mixture was warmed to room temperature and stirred for 100 minutes. Hydroquinone (0.18 g) was added thereto, and the mixture was heated while stirring at 60°C for 4 hours. After being allowed to cool, the resulting mixture was concentrated and distilled under reduced pressure to give a transparent liquid (22.2 g), which was further distilled under reduced pressure to give methacryloyl isocyanate as a fraction boiling at 52—53°C/4.42 kPa (39 mm Hg).

Example 2

A. To a solution of oxalyl chloride (243 g; 1.92 mol) in a 1,2 - dichloroethane (350 ml), a solution of methacrylamide (106 g; 1.25 mol) in 1,2 - dichloroethane (450 ml) was dropwise added over 50 minutes while ice-cooling at 0°C under a stream of nitrogen. The mixture was stirred at 0°C for 2 hours, and stirring was continued under reflux for 2 hours. After being allowed to cool, the resultant mixture was concentrated and distilled under reduced pressure to give methacryloyl isocyanate (63 g; yield, 45%) as a fraction boiling at 52—53°C/4.42 kPa (39 mm Hg) (transport liquid) and then α - methyl - β - chloropropionyl isocyanate (75.4 g; yield, 40.9%) as a fraction boiling at 83—85°C/4.42 kPa (39 mm Hg) (transparent liquid).

B. To a solution of α - methyl - β - chloropropionyl isocyanate (14.75 g; 100 mmol) in toluene (20 ml), molecular sieve 4A (20 g) was added, and the mixture was heated under reflux for 13.5 hours under a stream of nitrogen. After being allowed to cool, the molecular sieve was collected by filtration, and the filtrate was distilled under reduced pressure to give methyacryloyl isocyanate (3.55 g). In addition, some of the starting α - methyl - β - chloropropionyl isocyanate (3.54 g) was recovered.

When the procedure of Example 2B was repeated, but not using toluene, methacryloyl isocyanate was again obtained.

Example 3

A. To a solution of oxalyl chloride (95.25 g); 0.75 mol) in 1,2 - dichloroethane (150 ml), a solution of acrylamide (35.5 g; 0.5 mol) and hydroquinone (0.54 g) in 1,2 - dichloroethane (200 ml) was dropwise added over 30 minutes at −30 to 0°C under a stream of nitrogen. The mixture was heated and stirred under reflux for 1 hours. After being allowed to cool, the resultant mixture was distilled under reduced pressure to give β - chloropropionyl isocyanate (44.7 g) as a fraction boiling at 74—75°C/3.33 kPa (25 mm Hg) (transparent liquid).

B. The procedure of Example 2B was repeated, but using β - chloropropionyl isocyanate instead of α - methyl - β - chloropropionyl isocyanate. Acryloyl isocyanate was obtained, b.p. 82—83°C.

**Claims**

1. A process for preparing an alkenoyl isocyanate of the formula

$$CH_2{=}CR{-}CO{-}NCO \qquad (I:\ R{=}C_{1-5}\ alkyl)$$

which comprises reacting an acrylamide of the formula

$CH_2{=}CR{-}CONH_2$ (III: $R{=}C_{1-5}$ alkyl)

with an oxalyl halide of the formula

$(COX)_2$ (IV: X=halogen)

at −50 to +150°C; and recovering the alkenoyl isocyanate from the resultant reaction mixture by distillation.

2. A process according to claim 1, which comprises recovering separately from the reaction mixture a haloalkanoyl isocyanate of the formula

$XCH_2{-}CHR{-}CO{-}NCO$ (II: $R{=}C_{1-5}$ alkyl, X=halogen)

and reacting the isocyanate with a hydrogen halide-eliminating reagent at −50 to +200°C, to give the alkenoyl isocyanate (I: $R{=}C_{1-5}$ alkyl).

3. A process according to claim 1 or claim 2, wherein R is methyl.

4. A process for preparing acryloyl isocyanate (I: R=H), which comprises reacting acrylamide (III: R=H) with an oxalyl halide as defined in claim 1 (IV: X=halogen), at −50 to +150°C; and reacting the resultant β - halopropionyl isocyanate (II: R=H, X=halogen) with a hydrogen halide-eliminating reagent, at −50 to +200°C.

5. A process according to any preceding claim, which is conducted in the presence of a polymerisation inhibitor.

**Patentansprüche**

1. Verfahren zur Herstellung eines Alkenoylisocyanats der Formel

$CH_2{=}CR{-}CO{-}NCO$ (I: $R{=}C_{1-5}$-Alkyl)

welches die Umsetzung eines Acrylamids der Formel

$CH_2{=}CR{-}CONH_2$ (III: $R{=}C_{1-5}$-Alkyl)

mit einem Oxalylhalogenid der Formel

$(COX)_2$ (IV: X=Halogen)

bei −50 bis +150°C und die Gewinnung des Alkenoylisocyanats aus der resultierenden Reaktionsmischung durch Destillation umfaßt.

2. Verfahren nach Anspruch 1, welches die separate Gewinnung eines Halogenalkanoylisocyanats der Formel

$XCH_2{-}CHR{-}CO{-}NCO$ (II: $R{=}C_{1-5}$-Alkyl, X=Halogen)

aus der Reaktionsmischung und die Umsetzung des Isocyanats mit einem Mittel zur Entfernung des Wasserstoffhalogenids bei −50 bis +200°C zur Herstellung des Alkenoyliscyants (I: $R{=}C_{1-5}$-Alkyl) umfaßt.

3. Verfahren nach Anspruch 1 oder 2, worin R Methyl ist.

4. Verfahren zur Herstellung von Acryloylisocyanat (I: R=H), welches die Umsetzung von Acrylamid (III: R=H) mit einem Oxalylhalogenid wie in Anspruch 1 definiert (IV: X=Halogen) bei −50 bis +150°c und die Umsetzung des resultierenden β - Halogenpropionylisocyanats (II: R=H, X=Halogen) mit einem Reagens zur Entfernung des Wasserstoffhalogenids bei −50 bis +200°C umfaßt.

5. Verfahren nach einem der vorhergehenden Ansprüche, welches in Gegenwart eines Polymerisationsinhibitors durchgeführt wird.

**Revendications**

1. Procédé de préparation d'un alcénoyl isocyanate de formule

$CH_2{=}CR{-}CO{-}NCO$ (I: R=alcoyle en $C_1{-}C_5$)

dans lequel on fait réagir un acrylamide de formule

$CH_2{=}CR{-}CONH_2$ (III: R=alcoyle en $C_1{-}C_5$)

avec un halogénure d'oxalyle de formule

$$(COX)_2 \qquad \text{(IV: X=halogène)}$$

entre −50 et +150°C; et on recueille l'alcénoyl isocyanate à partir du mélange réactionnel résultant par distillation.

2. Procédé selon la revendication 1, dans lequel on recueille séparément à partir du mélange réactionnel un isocyanate d'haloalcanoyle de formule

$$XCH_2—CHR—CO—NCO \qquad \text{(II: R=alcoyle en } C_1—C_5 \text{ X=halogène)}.$$

et on fait réagir l'isocyanate avec un agent éliminant l'acide halohydrique entre −50 et +200°C, pour donner l'alcénoyl isocyanate (I:R=alcoyle en $C_1$—$C_5$).

3. Procédé selon la revendication 1 ou 2, dans lequel R est un méthyle.

4. Procédé de préparation d'isocyanate d'acryloyle (I: R=H), dans lequel on fait réagir de l'acrylamide (III: R=H) avec un halogénure d'oxalyle tel que défini dans la revendication 1 (IV: X=halogène), entre −50 et +150°C; et on fait réagir l'isocyanate de β - halopropionyle résultant (II: R=H, X=halogène) avec un agent éliminant l'acide halohydrique, entre −50 et +200°C.

5. Procédé selon l'une quelconque des revendications précédentes, que l'on conduit en présence d'un inhibiteur de polymérisation.